# FASCICULE DE BREVET EUROPEEN

(11) **EP 0 991 670 B1**
(45) Date de publication et mention de la délivrance du brevet: **31.10.2001**
(21) Numéro de dépôt: 98932194.8
(22) Date de dépôt: 12.06.1998
(51) Int. Cl.: C08B 37/16, B01J 20/24, C02F 1/28, A61K 31/715

(54) **FIXATION OU SEPARATION D'IONS, NOTAMMENT DE Pb, PAR DES DERIVES DE PER(3,6-ANHYDRO) CYCLODEXTRINES**
BINDUNG UND TRENNUNG VON IONEN, INSBESONDERE VON BLEI DURCH PER(3,6-ANHYDRO)CYCLODEXTRINDERIVATE
METHOD FOR FIXING OR SEPARATING IONS, IN PARTICULAR OF LEAD, USING PER(3,6-ANHYDRO) CYCLODEXTRIN DERIVATIVES

(30) Priorité: 13.06.1997 FR 9707339
(43) Date de publication de la demande: 12.04.2000
(73) Titulaire: COMMISSARIAT A L'ENERGIE ATOMIQUE, 75015 Paris (FR)
(72) Inventeur: BAUDIN, Cécile, F-75014 Paris (FR); PERLY, Bruno, F-78320 La Verrière (FR); GADELLE, Andrée, F-38830 Montbonnot (FR); DEBOUZY, Jean-Claude, F-38660 La Terrasse (FR); FAUVELLE, Florence, F-38000 Grenoble (FR)
(74) Mandataire: Des Termes, Monique
(86) Numéro de dépôt international: FR9801235
(87) Numéro de publication internationale: WO9856829

(56) Documents cités:
- H. YAMAMURA ET AL.: "A cyclodextrin derivative with cation carrying ability: Heptakis(3,6-anhydro)-beta-cyclodextrin 2-O-p-Phenylazobenzoate" CHEMISTRY LETTERS, vol. 9, 1996, pages 799-800, XP002055552 JP
- F. FAUVELLE ET AL.: "Letter: Electrospray ionisation and matrix-assisted laser desorption/ionisation mass spectrometric studies of cation complexation with per-3,6-anhydro-alpha-cyclodextrin" EUROPEAN MASS SPECTROMETRY, vol. 2, no. 6, 1996, pages 381-384, XP002055553
- P. R. ASHTON ET AL.: "A novel approach to the synthesis of some chemically modified cyclodextrins" J. ORG. CHEM., vol. 60, 1995, pages 3898-3903, XP002013616 USA cité dans la demande

## Description

### Domaine technique

La présente invention concerne un procédé de fixation ou de séparation d'ions, notamment de Pb, par des dérivés de per(3,6-anhydro)cyclodextrines.

### Etat de la technique antérieure

Les cyclodextrines ou cyclomaltooligosaccharides sont des composés d'origine naturelle formés par l'enchaînement d'unités glucose liés en α-(1,4).

De nombreux travaux ont montré que ces composés pouvaient former des complexes d'inclusion avec des molécules hydrophobes permettant ainsi leur solubilisation dans des milieux aqueux. De nombreuses applications ont été proposées pour tirer profit de ce phénomène, en particulier dans le domaine pharmaceutique, comme il est décrit par D. Duchêne "Pharmaceutical application of cyclodextrins" dans "Cyclodextrins and their industrial uses". D. Duchêne Ed., Editions de Santé, Paris, 1987, pp 213-257.

Des spécialités pharmaceutiques ont déjà été commercialisées au Japon, en Italie et plus récemment en France, sous forme de complexes dans les cyclodextrines. En France, le premier principe actif mis sur le marché sous la forme d'un complexe d'inclusion dans une cyclodextrine est le piroxicam, anti-inflammatoire commercialisé par Pierre Fabre Médicament, sous le nom de BREXIN®. Parmi les très nombreux dérivés modifiés de ces cyclodextrines, ceux pour lesquels la cavité est retournée sur elle-même présentent des propriétés intéressantes même si leur capacité à inclure des molécules organiques est perdue ou très limitée. Des composés de ce type sont les per(3,6-anhydro)cyclodextrines.

La synthèse de ces peranhydrocyclodextrines a été décrite dès 1991 dans le document 1 : Gadelle A. et Defaye J., Angew. Chem. Int. Ed. Engl., (1991), 30, 78-79 ; et le document 2 : Ashton P.R., Ellwood P., Staton I. and Stoddart J.F., Angew . Chem. Int. ed. Engl., (1991) 30, 80-81), et il a été montré que ces dérivés présentent des solubilités intéressantes aussi bien dans l'eau que dans les solvants organiques. Quelques études ultérieures (document 3 : Yamamura H. and Fujita K. Chem. Pharm. Bull., (1991) 39, 2505-2508 ; document 4 : Yamamura H., Ezuka T., Kawase Y., Kawai M., Butsugan Y. and Fujita K., J. Chem. Soc., Chem. Com., (1993) 636-637 ; et document 5 : Yamamura H. Nagaoka H., Kawai M. and Butsugan Y., Tetrahedron Lett. (1995) 36, 1093-1094) ont de plus montré que ces dérivés peranhydro pouvaient complexer des ions alcalins avec une sélectivité non négligeable.

Ashton et al dans J. Org. Chem., 60, 1995, p. 3898-3903 ont décrit la synthèse du dérivé de peranhydro-β-cyclodextrine substitué en position 2 par un groupe méthyle.

Toutefois, cette modification chimique n'a pas été effectuée en vue d'optimiser les propriétés de complexation ou de sélectivité des peranhydrocylodextrines.

### Exposé de l'invention

La présente invention a précisément pour objet l'utilisation pour la séparation ou la fixation d'ions de dérivés⁻ de peranhydrocyclodextrines dans lesquels une modification chimique a été effectuée pour modifier leurs propriétés, en particulier leur sélectivité vis-à-vis des ions qu'elles sont susceptibles de complexer, notamment vis-à-vis du plomb.

Selon l'invention, cette modification porte sur les groupes hydroxyle présents sur cette molécule ainsi que sur la configuration du carbone C₂ qui peut être inversée pour conduire à des dérivés de type L-mannose.

Aussi, l'invention a pour objet un procédé de fixation ou de séparation d'ions, qui consiste à mettre en contact un milieu contenant lesdits ions avec un dérivé de per (3,6-anhydro)- cyclodextrine répondant à l'une des formules suivantes : 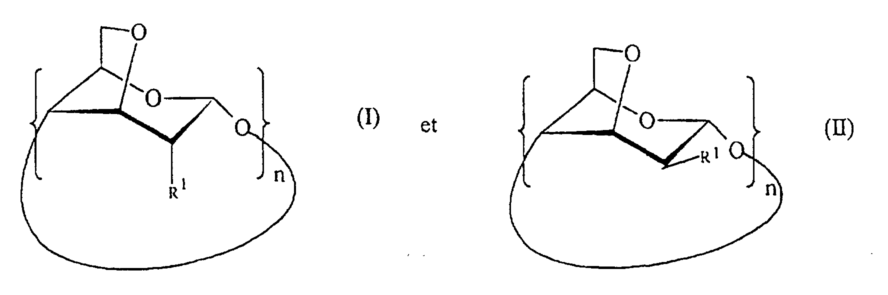 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6, 7 ou 8,
pour fixer lesdits ions sous forme de complexe avec le dérivé de per(3,6-anhydro)cyclodextrine et les séparer dudit milieu.

Dans le dérivé de cyclodextrine de formule (I) ou (II), les groupes hydrocarbonés aliphatiques ou aromatiques, susceptibles d'être utilisés pour R² et R³ peuvent être de divers types. Ils sont constitués par une chaîne carbonée dans laquelle certains atomes de carbone peuvent être remplacés par un ou plusieurs hétéroatomes tels que 0, S et N, et ils peuvent comporter une ou plusieurs insaturations éthyléniques ou acétyléniques. Par ailleurs, le groupe hydrocarboné peut comporter différents substituants, en particulier des groupes fonctionnels ou des atomes d'halogènes. Les groupes hydrocarbonés aromatiques peuvent être constitués par le groupe phényle et le groupe tosyle, éventuellement substitués, par exemple par des groupes alkyle de 1 à 20 atomes de carbone.

R² et R³ peuvent en particulier représenter un groupe alkyle linéaire ou ramifié de 1 à 20 atomes de carbone.

Selon un mode de réalisation préféré de l'invention, destiné notamment à la séparation d'ions plomb, le dérivé de per(3,6-anhydro)cyclodextrine est un dérivé d'α-cyclodextrine, c'est-à-dire que dans les formules (I) et (II) données ci-dessus, n est égal à 6.

De préférence encore, le dérivé utilisé répond à la formule (I) dans laquelle tous les R¹ représentent le groupe méthoxy et n est égal à 6.

Les ions susceptibles d'être fixées ou séparés par le procédé de l'invention peuvent être de divers types ; il peut s'agir par exemple d'ions de métaux alcalins, d'actinides, de lanthanides ou de métaux polluants tels que le plomb, le mercure, le cobalt et le strontium.

Le procédé de l'invention s'applique en particulier à la séparation et à la fixation du plomb sous forme de complexe.

En effet, le plomb et ses dérivés polluent l'environnement et sont toxiques aussi bien chez l'animal que chez l'homme. Les principaux effets toxiques affectent le développement neurologique et le fonctionnement du système nerveux. Il est donc nécessaire de séparer et d'éliminer le plomb de l'environnement et de le stocker de manière sûre.

Par ailleurs, des produits qui permettraient d'assurer la décontamination en plomb des êtres vivants en empêchant l'action du plomb sur le système nerveux et sur d'autres organes, seraient d'un grand intérêt pour résoudre ces problèmes.

Selon l'invention, on a trouvé que les dérivés des per(3,6-anhydro)cyclodextrines répondant aux formules (I) et (II) données ci-dessus, présentaient une spécificité élevée pour le plomb et étaient capables de complexer celui-ci avec des rendements élevés pouvant atteindre 100 %, même en présence d'autres ions tels que les ions sodium.

De cette façon, on peut séparer le plomb du milieu environnant sous la forme de complexe.

Aussi, l'invention a également pour objet les complexes de plomb et de dérivés de per(3,6-anhydro)cyclodextrines de formule (I) ou (II) décrits ci-dessus.

Pour mettre en oeuvre le procédé de l'invention, on peut utiliser le dérivé de per(3,6-anhydro)cyclodextrine de formule (I) ou (II) sous forme de solution aqueuse ou de solution organique.

Lorsque le milieu contenant les ions à séparer ou à fixer est une solution aqueuse, on peut dissoudre le dérivé de cyclodextrine dans un solvant organique immiscible avec la solution aqueuse, par exemple dans du chloroforme, pour former le complexe dans la solution organique et le séparer facilement de la solution aqueuse.

On peut aussi utiliser le dérivé de cyclodextrine en solution aqueuse, notamment pour assurer la décontamination en plomb d'êtres vivants.

En effet, on sait que les dérivés de cyclodextrines de formule (I) ou (II) sont des composés biocompatibles. Ils peuvent donc être administrés à l'homme ou à l'animal pour assurer la fixation du plomb sous forme de complexe et éviter ainsi son interaction avec les organes du corps humain ou animal.

Aussi, l'invention a également pour objet une composition pharmaceutique pour la décontamination en plomb d'un être vivant, caractérisée en ce qu'elle comprend un dérivé de per(3,6-anhydro)cyclodextrine répondant à l'une des formules suivantes : 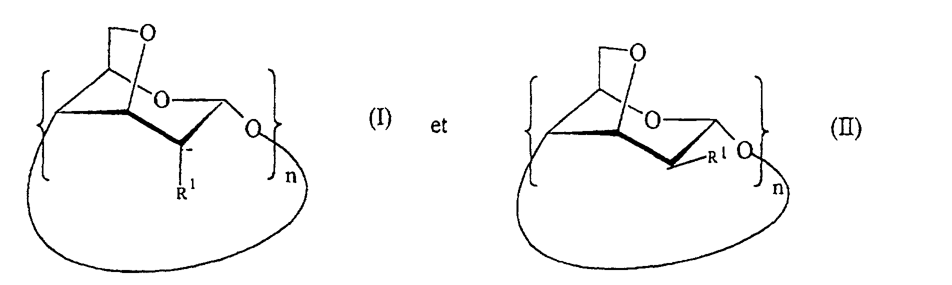 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi 0, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6, 7 ou 8.

De préférence, le dérivé de per(3,6-anhydro)cyclodextrine utilisé dans cette composition répond à la formule (I) dans laquelle tous les R¹ représentent le groupe méthoxy et n est égal à 6.

Cette composition peut être administrée par voie orale ou par injection.

Les solutions aqueuses peuvent comprendre jusqu'à 0,08 mol/l de dérivé de formule (I).

Les quantités administrées dépendront du taux de contamination par le plomb et du poids du patient.

L'invention a encore pour objet les dérivés de per(3,6-anhydro)cyclodextrines, utilisables dans ce procédé, qui répondent à l'une des formules suivantes : 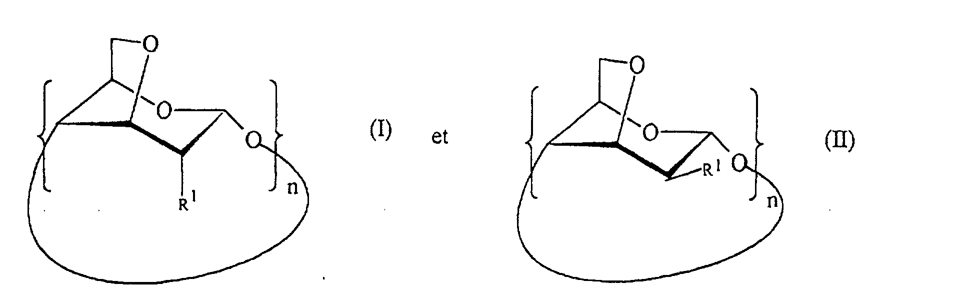 dans lesquelles l'un au moins des R¹ représente le groupe méthoxy et les autres R¹ qui peuvent être identiques ou différents, représentent un atome d'hydrogène, un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6,7 ou 8 à condition que tous les R¹ ne représentent pas OCH₃ lorsque n = 7 et que le dérivé répond à la formule (I).

Les dérivés de cyclodextrine utilisés dans l'invention peuvent être préparés par différents procédés.

Lorsque le dérivé de cyclodextrine répond à la formule (I) ou (II) donnée ci-dessus dans laquelle au moins l'un des R¹ représente le groupe méthoxy, les autres R¹ représentant OH ou OCH₃ et n étant égal à 6, 7 ou 8, ceux-ci peuvent être préparés par un procédé comprenant les étapes suivantes :
- 1⁻) faire réagir une peranhydrocyclodextrine répondant à l'une des formules : 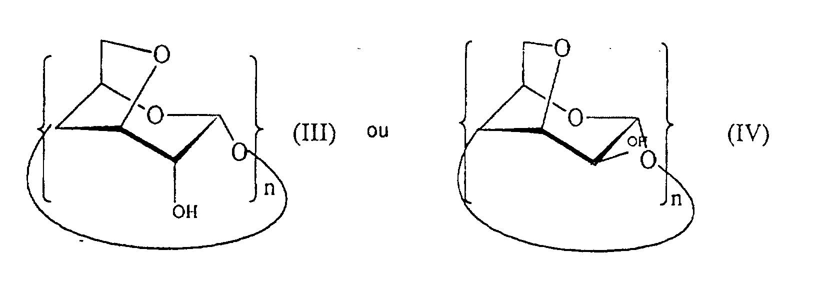 dans lesquelles n est égal à 6, 7 ou 8,
   avec un hydrure de métal alcalin pour convertir le(s) groupe(s) OH en groupe(s) OM avec M représentant un métal alcalin ;
- 2) faire réagir la peranhydrocyclodextrine modifiée obtenue en 1) avec un halogénure de méthyle de formule CH₃X dans laquelle X représente un atome d'halogène ; et
- 3) faire réagir si nécessaire la peranhydrocyclodextrine obtenue en 2) avec un ou plusieurs réactifs pour la substituer par des groupes R¹ différents de OCH₃.

Pour effectuer l'étape 2), on utilise la quantité nécessaire de CH₃X pour modifier un ou plusieurs des groupe OH de la cyclodextrine.

Lorsque le dérivé de cyclodextrine répond à la formule (I) ou (II) donnée ci-dessus dans laquelle les autres R¹ représentent OR² avec R² ayant la signification donnée ci-dessus sauf CH₃, on procède comme précédemment pour introduire le (s) groupe(s) OCH₃, puis on fait réagir ensuite le dérivé avec un halogénure de formule R²X dans laquelle R² a la signification donnée ci-dessus et X est un atome d'halogène.

Lorsque le dérivé de cyclodextrine répond à la formule (I) ou (II) dans laquelle les autres R¹ représentent OCOR², on procède comme précédemment pour introduire tout d'abord les groupes méthoxy, puis on fait réagir ensuite le dérivé méthylé avec un halogénure ou anhydride d'acide de formules R²COX ou (R²CO)₂O dans lesquelles R2 a la signification donnée ci-dessus et X représente un atome d'halogène, pour remplacer les hydroxyles restants par OCOR².

Lorsque l'on veut préparer un dérivé de cyclodextrine dans lequel le(s) autre(s) R¹ représentent un atome d'halogène ou un groupe de formule SH, SR², NH₂, NR²R³, CONR²R³, CONH₂, CN, COOR², COOH, ou R², avec R² et R³ ayant les significations données ci-dessus, et n est égal à 6, 7 ou 8, on peut effectuer les étapes suivantes en partant d'une peranhydrocyclodextrine partiellement méthylée, c'est-à-dire dans laquelle l'un au moins des R¹ représente OCH₃ et les autres R¹ représentent OH, et en effectuant les étapes suivantes :
1) faire réagir cette peranhydrocyclodextrine avec un hydrure de métal alcalin pour convertir le (s) groupe(s) OH en groupe(s) OM avec M représentant un métal alcalin ;
2) faire réagir la peranhydrocyclodextrine modifiée obtenue en 1) avec un chlorure de formule ClSO₂R² avec R² ayant la signification donnée ci-dessus, pour obtenir le dérivé de formule (I) ou (II) dans laquelle l'un au moins des R¹ est un groupe de formule OSO₂R² ; et
3) faire réagir le dérivé obtenu dans la deuxième étape avec un ou plusieurs réactifs appropriés pour remplacer OSO₂R² par le groupe R¹ voulu.

Dans ce procédé on transforme tout d'abord la per(3,6-anhydro)cyclodextrine en alcoolate par action d'hydrure de métal alcalin, puis on convertit cet alcoolate en dérivé comportant un groupe partant de formule OSO₂R², que l'on fait réagir ensuite en une ou plusieurs étapes avec un ou plusieurs réactifs appropriés pour remplacer ce groupe partant par le groupe R¹ voulu.

Ainsi, dans le cas où R¹ doit représenter NH₂, on peut faire réagir N₃M et le composé défini en 2). Le composé ainsi obtenu appelé azide peut subir une hydrogénation catalytique ou être traité en présence d'ammoniac NH₃, afin d'obtenir le produit où R¹ doit représenter NH₂.

Le produit où R¹ doit représenter NR²R³ est obtenu en faisant réagir le composé défini en 2) sur le composé NHR²R³.

Dans le cas où R¹ doit représenter SH ou SR², on peut faire réagir le composé défini en 2) avec un halogénure X⁻, ce qui donne le composé avec (R¹ = X), que l'on fait ensuite réagir avec HS⁻ ou R²S⁻ pour donner un composé où R¹ représentera SH ou SR².

Lorsque R¹ doit représenter un groupe hydrocarboné, on fait réagir avec R¹₂LiCu (R¹ représente un groupe hydrocarboné) pour donner un composé final où R¹ représente alors un groupe hydrocarboné.

De même, le composé où R¹ représente un halogène peut réagir avec CN⁻ pour donner un composé final où R¹ représentera CN.

De même, le composé où R¹ représente CN peut par hydrolyse ménagée donner un composé où R¹ représentera CONH₂. Le composé où R¹ représente CN peut par hydrolyse complète donner un composé où R¹ représentera COOH.

Le composé où R¹ représente COOH peut par estérification donner un composé où R¹ représentera COOR².

Le composé où R¹ représente COOH peut réagir sur NHR²R³ en présence de DCC (dicyclohexylcarbodiimide) pour donner un composé où R¹ représentera NR²R³.

Les dérivés de cyclodextrine de l'invention présentent de nombreux avantages. En particulier lorsqu'ils sont persubstitués, c'est-à-dire lorsque tous les R¹ sont différents du groupe OH, on a des dérivés qui présentent une bonne solubilité dans les solvants organiques tels que le chloroforme, l'acétone, le tétrahydrofurane etc. Cette solubilité est intéressante pour leur utilisation dans la séparation ionique car elle permet de réaliser la séparation par des procédés d'échanges liquide-liquide qui sont bien connus dans la technique.

Par ailleurs, la possibilité d'introduire un ou plusieurs groupes chimiques particuliers permet de construire sur mesure des agents complexants pour des ions très divers. Cette facilité est de plus amplifiée par le fait que les trois cyclodextrines naturelles qui peuvent être utilisées comme matière de base, ont des diamètre de cavité différents qui peuvent apporter une sélection supplémentaire en rapport avec la taille des ions à séparer.

Les produits de départ de formules (III) ou (IV) utilisés dans l'invention peuvent être préparés par des procédés classiques tels que ceux décrits dans les documents 1 et 2 précités de Gadelle A. et al. et de Ashthon P. R. et al.

D'autres caractéristiques et avantages de l'invention apparaîtront mieux à la lecture des exemples qui suivent, donnés à titre illustratif et non limitatif en référence au dessin annexé.

### Brève description du dessin

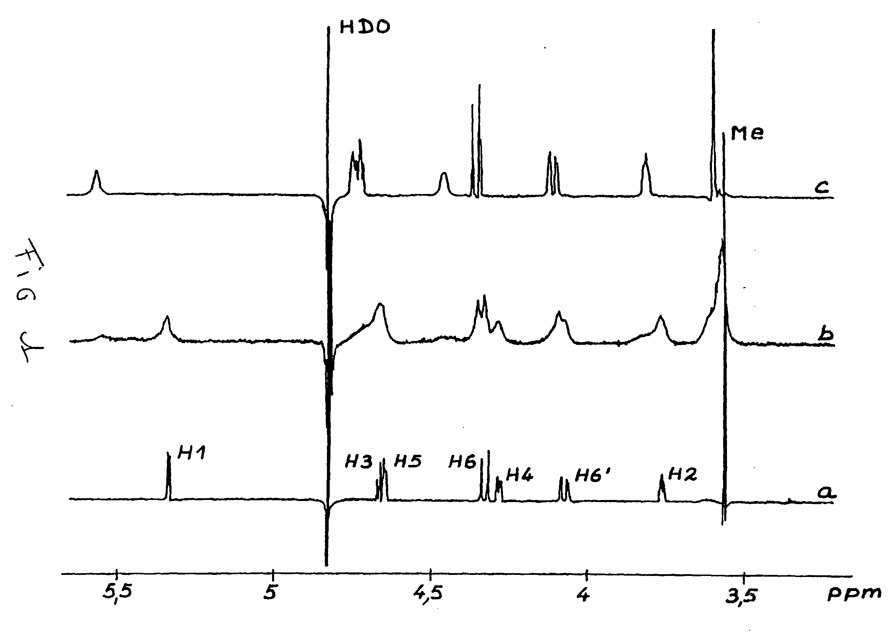

Les figures 1(a), 1(b) et 1(c) sont des spectres de résonance magnétiques nucléaire (RMN) du proton du dérivé de l'exemple 1 seul (a), ou en présence de 1 mmol/L de nitrate de plomb ou en présence de 3 mmol/L de nitrate de plomb.

### Exposé détaillé des modes de réalisation

### Exemple 1 : Préparation de l'hexakis (3,6-anhydro-2-0-méthyl) cyclomaltohexaose.

Ce composé répond à la formule (I) donnée ci-dessus dans laquelle tous les R¹ représentent OCH₃ et n est égal à 6.

On pèse 50 mg (0,057 mmol) d'hexakis (3,6-anhydro)cyclomaltohexaose séché sous vide à 120°C pendant 48 heures, on y ajoute 10 ml de diméthylformamide (DMF) anhydre et on chauffe la solution pendant 15 minutes à 70°C jusqu'à l'obtention d'une dispersion. Dans un autre ballon, on pèse 82 mg d'hydrure de sodium dispersés dans l'huile auxquels on ajoute 10 ml de DMF anhydre. Sous agitation, on ajoute dans ce dernier ballon à la seringue la suspension d'hexakis(3,6-anhydro)cyclomaltohexaose. Après 25 minutes d'agitation, on additionne à la seringue 200 µl d'iodure de méthyle CH₃I(3 mmol). Après 15 minutes d'agitation, on évapore le solvant et on dissout le résidu dans l'eau. La solution est lavée au chloroforme afin d'éliminer les huiles. On lyophilise la partie aqueuse. Celle-ci est chromatographiée sur la colonne PBMN polyamine fabriquée par Y.M.C. en utilisant un gradient 0 à 30 % d'eau dans l'acétonitrile, et caractérisée par résonance magnétique nucléaire du proton à 500 MHz, à une température de 298 K et à une concentration de 3 mmol/L dans D₂O.

Les résultats obtenus sont représentés sur la figure 1(a).

### Exemple 2 : Formation de complexe de plomb

On ajoute à une solution aqueuse du dérivé perméthylé obtenu dans l'exemple 1, une solution aqueuse de nitrate de plomb de façon à obtenir une solution aqueuse comprenant 3 mmol/L de dérivé perméthylé et 1 mmol/L de nitrate de plomb. On analyse la solution obtenue par résonance magnétique nucléaire dans les mêmes conditions que celles de l'exemple 1 (500 MHz, D₂O, 298K).

Les résultats obtenus sont données sur la figure 1 (b).

Dans ce cas, l'échange est suffisamment lent par rapport au temps d'observation de la RMN pour observer les deux signaux correspondant à la cyclodextrine libre (figue 1(a)) et au complexe (figure 1(b)). Les surfaces respectives des parties libre et complexée représentent 2 pour 1, ce qui signifie que tout le plomb présent est complexé.

Sur la figure 1(b), une séparation nette entre la cyclodextrine libre et complexée est visible pour le proton H₁ seulement. L'élargissement des signaux est caractéristique d'un échange lent.

### Exemple 3 : Formation d'un complexe de plomb.

On suit le même mode opératoire que dans l'exemple 2, mais la solution aqueuse comprend 3 mmol/L du dérivé perméthylé de l'exemple 1 et 3 mmol/L de nitrate de plomb.

Le spectre RMN du proton est représenté sur la figure 1 (c).

De même, dans ce cas, seuls les signaux du complexe sont visibles. Un seul signal élargi est observé pour le H1. Un tel comportement est observable pour une constante d'affinité extrêmement élevée.

### Exemple 4 : Formation du complexe de plomb

On suit le même mode opératoire que dans l'exemple 3, mais on ajoute de plus du nitrate de sodium de façon à obtenir une solution aqueuse comprenant :
- 3 mmol/L du dérivé perméthylé de l'exemple 1.
- 3 mmol/L de nitrate de plomb, et
- 3 mmol/L de nitrate de sodium.

Le spectre de RMN du complexe obtenu est identique à celui représenté sur la figure 1 (c).

Ainsi, le spectre RMN du complexe n'est pas modifié par la présence de nitrate de sodium 3mM. Ce résultat est de toute première importance en vue d'applications dans le domaine biologique car il montre que le plomb peut être complexé même en présence d'un excès d'ions sodium.

Les -résultats de RMN obtenus dans les exemples 2 à 4 montrent que la constante d'affinité du dérivé perméthylé de l'exemple 1 pour le Pb est très supérieure à celle que l'on obtient avec l'hexakis (3,6-anydro)cyclomaltohexaose de départ, celle-ci étant de l'ordre de 10⁵ dans le cas du dérivé perméthylé et de l'ordre de 2500 dans le cas de la per(anhydro)cyclodextrine de départ.

Ce dérivé perméthylé est donc très intéressant, notamment pour la décontamination du plomb chez les êtres vivants.

En effet, il n'est ni toxique, ni hémolytique, alors que la per(3,6-anhydro)cyclodextrine non méthylée correspondante est hémolytique. Par ailleurs, il peut complexer le plomb même en présence de teneurs importantes de sodium.

### LISTE DES DOCUMENTS CITES

- Document 1 :: Gadelle A. et Defaye J., Angew. Chem. Int. Ed. Engl., 1991, 30, pages 79-79.
- Document 2 :: Ashton P.R., Ellwood P., Staton I and Stoddart J.F., Angew. Chem. Int. ed. Engl., 1991, 30, page 80-81.
- Document 3 :: Yamamura H. and Fujita K., Chem. Pharm. Bull., 1991, 39, pages 2505-2508.
- Document 4 :: Yamamura H., Esuka T., Kawase Y., Kawai M., Butsugan Y. and Fujita K., J. Chem. Soc., Chem. Commun., 1993, pages 636-637.
- Document 5 :: Yamamura H., Nagaoka H., Kawai M and Butsugan Y., Tetrahedron Lett., 1995, 3b, pages 1093-1094.
- Document 6 :: Ashton et al, J. Org. Chem., 1995, 60, pages 3898-3903.

## Revendications

1. Procédé de fixation ou de séparation d'ions consistant à mettre en contact un milieu contenant lesdits ions avec un dérivé de per (3,6-anhydro)cyclodextrine répondant à l'une des formules suivantes : 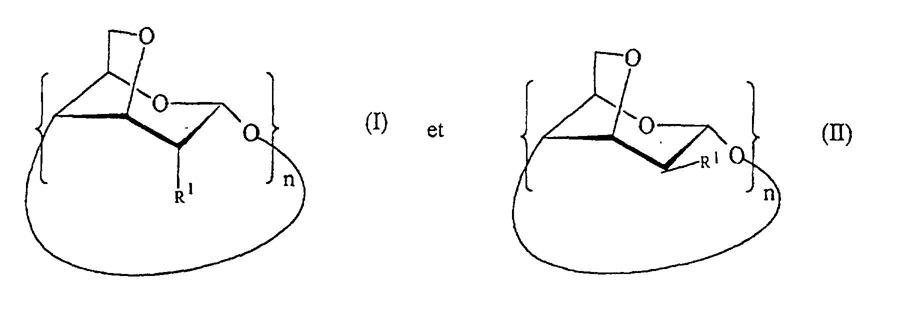 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6, 7 ou 8,
pour fixer lesdits ions sous forme de complexe avec le dérivé de per(3,6-anhydro)cyclodextrine et les séparer dudit milieu.

2. Procédé selon la revendication 1 dans lequel lesdits ions sont des ions de plomb.

3. Procédé selon la revendication 1 ou 2 dans lequel n est égal à 6.

4. Procédé selon la revendication 1 ou 2 dans lequel le dérivé de per(3,6-anhydro)cyclodextrine répond à la formule (I) dans laquelle tous les R¹ représentent le groupe méthoxy et n est égal à 6.

5. Procédé _ selon l'une quelconque des revendications 1 à 4, dans lequel ledit milieu étant une solution aqueuse, le dérivé de cyclodextrine est dissous dans un solvant organique immiscible avec la solution aqueuse.

6. Composition pharmaceutique pour la décontamination en plomb d'un être vivant, **caractérisée en ce qu'**elle comprend un dérivé de per(3,6-anhydro)cyclodextrine répondant à l'une des formules suivantes : 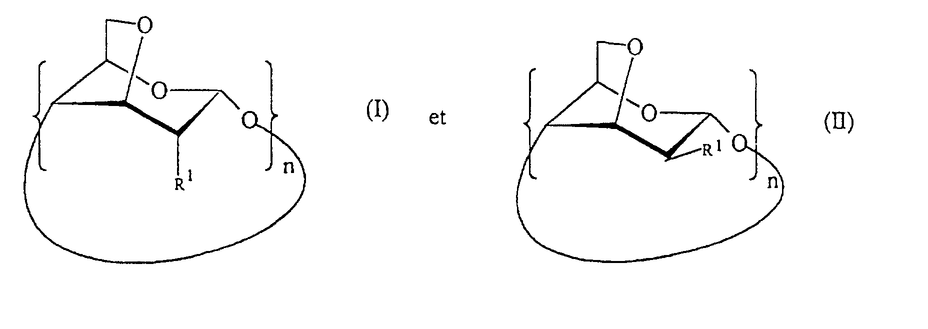 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6, 7 ou 8.

7. Composition pharmaceutique selon la revendication 6, dans laquelle le dérivé de per(3,6-anhydro)cyclodextrine répond à la formule (I) dans laquelle tous les R¹ représentent le groupe méthoxy et n est égal à 6.

8. Complexe de plomb et d'un dérivé de per (3,6-anhydro)cyclodextrine répondant à l'une des formules suivantes. 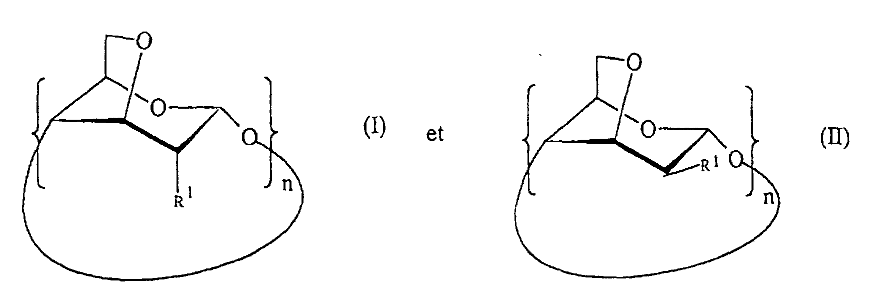 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6, 7 ou 8.

9. Complexe selon la revendication 8, dans lequel n est égal à 6.

10. Complexe selon la revendication 8, dans lequel le dérivé de per(3,6-anhydro) cyclodextrine répond à la formule (I) dans laquelle tous les R¹ représente le groupe méthoxy et n est égal à 6.

11. Dérivé de per(3,6 anhydro)cyclodextrine répondant à l'une des formules suivantes : 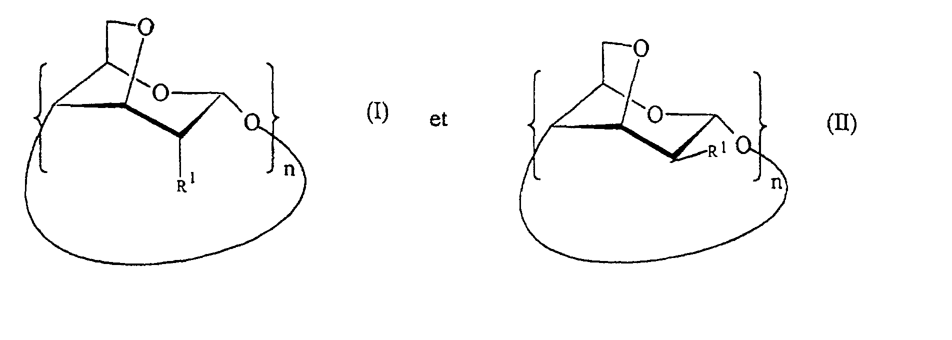 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6,7 ou 8 à condition que tous les R¹ ne représentent pas OCH₃ lorsque n = 7 et que le dérivé répond à la formule (I).

12. Dérivé de per(3,6-anhydro)cyclodextrine selon la revendication 11 répondant à la formule (I) dans laquelle tous les R¹ représentent le groupe OCH₃ et n est égal à 6.

13. Procédé de préparation d'un dérivé de per(3,6-anhydro)cyclodextrine de formule (I) ou (II) : 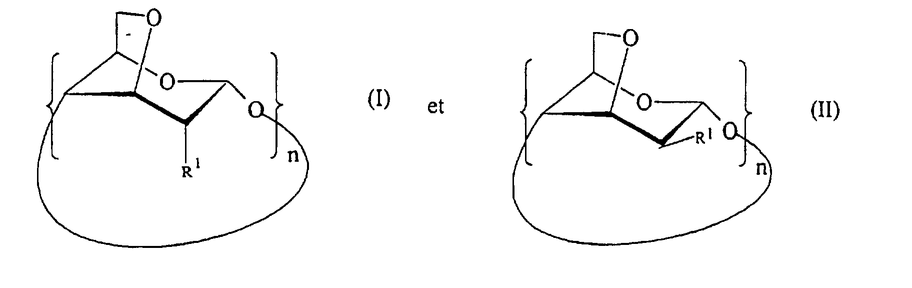 dans lesquelles l'un au moins des R¹ représente le groupe méthoxy et les autres R¹ qui peuvent être identiques ou différents, représentent un groupe répondant à l'une des formules : OH, OR², SH, SR², OCOR², NH₂, NR²R³, CONR²R³, CONH₂, CN, COOR², COOH et R², dans lesquelles R² représente un groupe hydrocarboné, aliphatique ou aromatique, saturé ou insaturé, pouvant comporter un ou plusieurs hétéroatomes choisis parmi O, S et N, et R³ représente un atome d'hydrogène ou un groupe hydrocarboné, aliphatique ou aromatique, saturé ou insaturé, pouvant comporter un ou plusieurs hétéroatomes choisis parmi O, S et N, et n est égal à 6, 7 ou 8, comprenant les étapes suivantes :
- 1) faire réagir une peranhydrocyclodextrine répondant à l'une des formules : 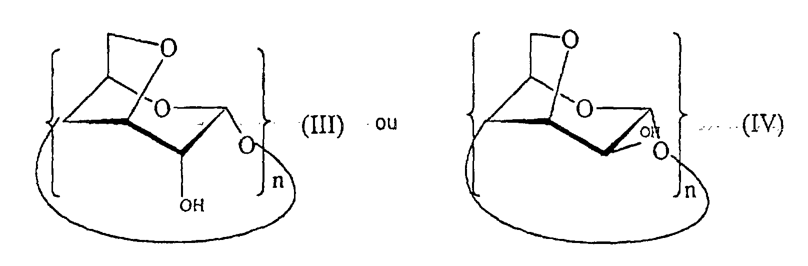 dans lesquelles n est égal à 6, 7 ou 8,
avec un hydrure de métal alcalin pour convertir le(s) groupe (s) OH en groupe(s) OM avec M représentant un métal alcalin ;
- 2) faire réagir la peranhydrocyclodextrine modifiée obtenue en 1) avec un halogénure de méthyle de formule CH₃X dans laquelle X représente un atome d'halogène ; et
- 3) faire réagir si nécessaire la peranhydrocyclodextrine obtenue en 2) avec un ou plusieurs réactifs pour la substituer par des groupes R¹ différents de OCH₃.

## Patentansprüche

1. Verfahren zur Bindung oder Trennung von lonen, darin bestehend, ein die genannten lonen enthaltendes Medium in Kontakt zu bringen mit einem Per(3,6-Anhydro)Cyclodextrin-Derivat, das einer der folgenden Formeln entspricht: 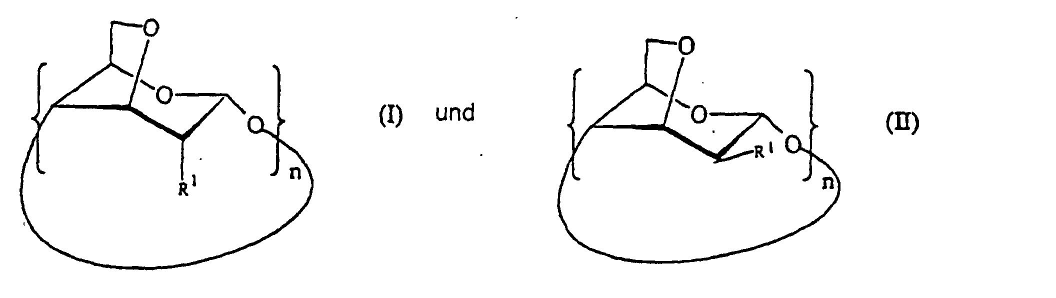 in denen wenigstens eines der R¹ die Methoxy-Gruppe bildet und die anderen R¹, die identisch oder unterschiedlich sein können, eine Gruppe bilden, die einer der folgenden Formeln entspricht: OH, OR², SH, SR², OCOR², NH₂, NR²R³, CONR²R³, CONH₂, CN, COOR², COOH und R², in denen R² eine kohlenwasserstoffhaltige, aliphatische oder aromatische Gruppe bildet, gesättigt oder ungesättigt, die ein oder mehrere Heteroatome umfassen kann, ausgewählt unter O, S und N, und R³ ein Wasserstoffatom darstellt oder eine kohlenwasserstoffhaltige, aliphatische oder aromatische Gruppe, gesättigt oder ungesättigt, die ein oder mehrere Heteroatome umfassen kann, ausgewählt unter O, S und N, und n gleich 6, 7 oder 8 ist,
um die genannten lonen durch Komplexbildung an das Per(3,6-Anhydro)Cyclodextrin-Derivat zu binden und von dem genannten Medium zu trennen.

2. Verfahren nach Anspruch 1, bei dem die genannten Ionen Blei-Ionen sind.

3. Verfahren nach Anspruch 1 oder 2, bei dem n gleich 6 ist.

4. Verfahren nach Anspruch 1 oder 2, bei dem das Per(3,6-Anhydro)Cyclodextrin-Derivat der Formel (I) entspricht, in der alle R¹ die Methoxy-Gruppe bilden und n gleich 6 ist.

5. Verfahren nach einem der Ansprüche 1 bis 4, bei dem das genannte Medium eine wässrige Lösung ist, das Cyclodextrin-Derivat in einem mit der wässrigen Lösung nicht mischbaren organischen Lösungsmittel aufgelöst ist.

6. Pharmazeutische Zusammensetzung zur Blei-Entgiftung eines Lebewesens,
**dadurch gekennzeichnet,**
**dass** sie ein Per(3,6-Anhydro)Cyclodextrin-Derivat umfasst, das einer der folgenden Formeln entspricht: 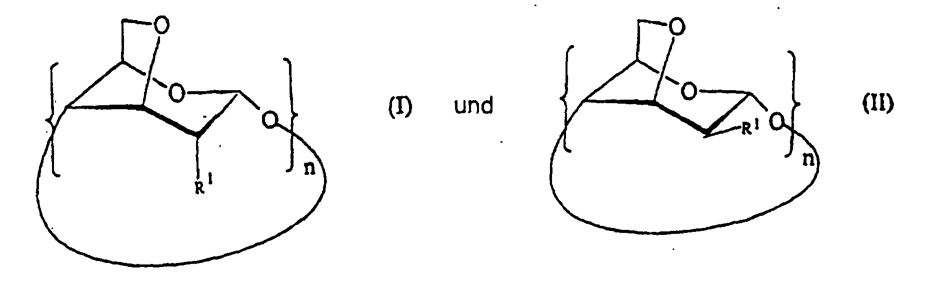 in denen wenigstens eines der R¹ die Methoxy-Gruppe bildet und die anderen R¹, die identisch oder unterschiedlich sein können, eine Gruppe bilden, die einer der folgenden Formeln entspricht: OH, OR², SH, SR², OCOR², NH₂, NR²R³, CONR²R³, CONH₂, CN, COOR², COOH und R², in denen R² eine kohlenwasserstoffhaltige, aliphatische oder aromatische Gruppe bildet, gesättigt oder ungesättigt, die ein oder mehrere Heteroatome umfassen kann, ausgewählt unter O, S und N, und R³ ein Wasserstoffatom darstellt oder eine kohlenwasserstoffhaltige, aliphatische oder aromatische Gruppe, gesättigt oder ungesättigt, die ein oder mehrere Heteroatome umfassen kann, ausgewählt unter O, S und N, und n gleich 6, 7 oder 8 ist,
um die genannten lonen durch Komplexbildung an das Per(3,6-Anhydro)Cyclodextrin-Derivat zu binden und von dem genannten Medium zu trennen.

7. Pharmazeutische Zusammensetzung nach Anspruch 6, bei dem das Per(3,6-Anhydro)Cyclodextrin-Derivat der Formel (I) entspricht, in der alle R¹ die Methoxy-Gruppe bilden und n gleich 6 ist.

8. Komplex aus Blei und einem Per(3,6-Anhydro)Cyclodextrin-Derivat, das einer der folgenden Formeln entspricht: 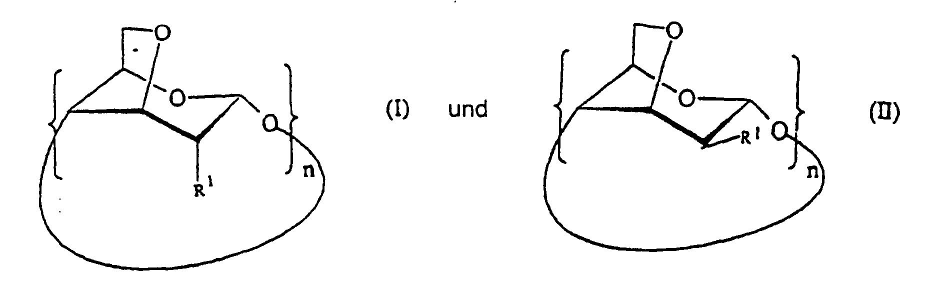 in denen wenigstens eines der R¹ die Methoxy-Gruppe bildet und die anderen R¹, die identisch oder unterschiedlich sein können, eine Gruppe bilden, die einer der folgenden Formeln entspricht: OH, OR², SH, SR², OCOR², NH₂, NR²R³, CONR²R³, CONH₂, CN, COOR², COOH und R², in denen R² eine kohlenwasserstoffhaltige, aliphatische oder aromatische Gruppe bildet, gesättigt oder ungesättigt, die ein oder mehrere Heteroatome umfassen kann, ausgewählt unter O, S und N, und R³ ein Wasserstoffatom darstellt oder eine kohlenwasserstoffhaltige, aliphatische oder aromatische Gruppe, gesättigt oder ungesättigt, die ein oder mehrere Heteroatome umfassen kann, ausgewählt unter O, S und N, und n gleich 6, 7 oder 8 ist.

9. Komplex nach Anspruch 8, bei dem n gleich 6 ist.

10. Komplex nach Anspruch 8, bei dem das Per(3,6-Anhydro)Cyclodextrin-Derivat der Formel (I) entspricht, in der alle R¹ die Methoxy-Gruppe bilden und n gleich 6 ist.

11. Per(3,6-Anhydro)Cyclodextrin-Derivat, das einer der folgenden Formeln entspricht: 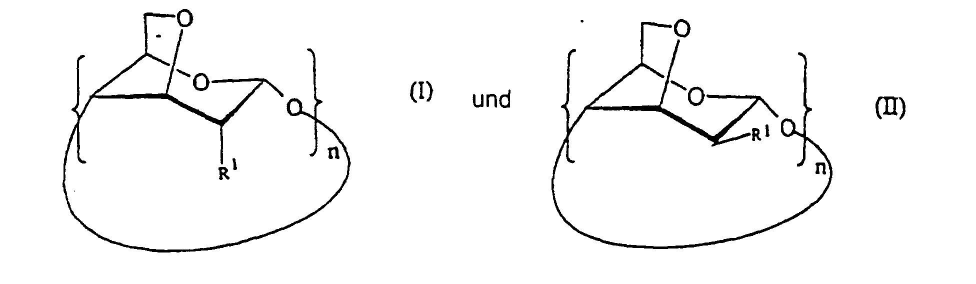 in denen wenigstens eines der R¹ die Methoxy-Gruppe bildet und die anderen R¹, die identisch oder unterschiedlich sein können, eine Gruppe bilden, die einer der folgenden Formeln entspricht: OH, OR², SH, SR², OCOR², NH₂, NR²R³, CONR²R³, CONH₂, CN, COOR², COOH und R², in denen R² eine kohlenwasserstoffhaltige, aliphatische oder aromatische Gruppe bildet, gesättigt oder ungesättigt, die ein oder mehrere Heteroatome umfassen kann, ausgewählt unter O, S und N, und R³ ein Wasserstoffatom darstellt oder eine kohlenwasserstoffhaltige, aliphatische oder aromatische Gruppe, gesättigt oder ungesättigt, die ein oder mehrere Heteroatome umfassen kann, ausgewählt unter O, S und N, und n gleich 6, 7 oder 8 ist, unter der Bedingung, dass alle R¹ nicht OCH₃ darstellen, wenn n = 7 ist, und dass das Derivat der Formel (I) entspricht.

12. Per(3,6-Anhydro)Cyclodextrin-Derivat nach Anspruch 11, der Formel (I) entspricht, in der alle R¹ die OCH₃-Gruppe bilden und n gleich 6 ist.

13. Verfahren zur Herstellung eines Per(3,6-Anhydro)Cyclodextrin-Derivats der Formel (I) oder (II): 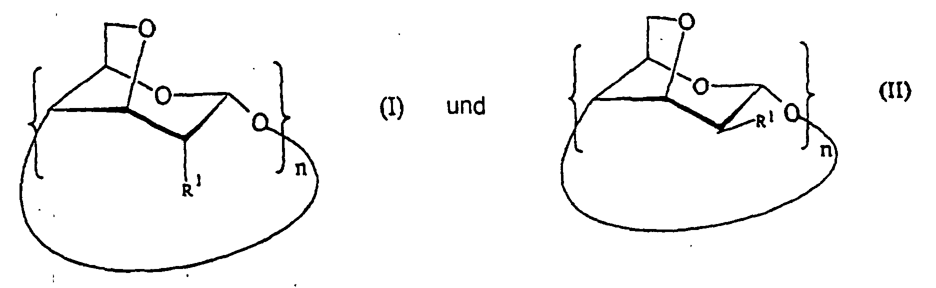 in denen wenigstens eines der R¹ die Methoxy-Gruppe bildet und die anderen R¹, die identisch oder unterschiedlich sein können, eine Gruppe bilden, die einer der folgenden Formeln entspricht: OH, OR², SH, SR², OCOR², NH₂, NR²R³, CONR²R³, CONH₂, CN, COOR², COOH und R², in denen R² eine kohlenwasserstoffhaltige, aliphatische oder aromatische Gruppe bildet, gesättigt oder ungesättigt, die ein oder mehrere Heteroatome umfassen kann, ausgewählt unter O, S und N, und R³ ein Wasserstoffatom darstellt oder eine kohlenwasserstoffhaltige, aliphatische oder aromatische Gruppe, gesättigt oder ungesättigt, die ein oder mehrere Heteroatome umfassen kann, ausgewählt unter O, S und N, und n gleich 6, 7 oder 8 ist,
die folgenden Schritte umfassend:
- 1) man lässt ein Peranhydrocyclodextrin, das einer der folgenden Formeln entspricht: 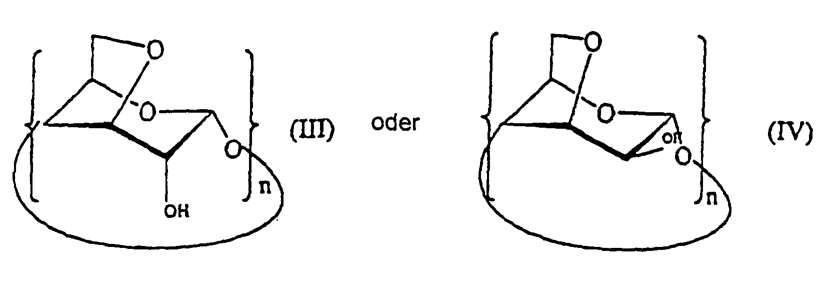 in denen n gleich 6, 7 oder 8 ist,
mit einem Alkalimetallhydrid reagieren, um die OH-Gruppe(n) umzuwandeln in eine OM-Gruppe bzw. OM-Gruppen, wobei M ein Alkalimetall darstellt;
- 2) man lässt das in 1) erhaltene modifizierte Peranhydrocyclodextrin mit einem Methylhalogenid der Formel CH₃X reagieren, in dem X ein Halogenatom darstellt; und
- 3) man lässt, wenn nötig, das in 2) erhaltene Peranhydrocyclodextrin mit einem oder mehreren Reagenzien reagieren, um es durch R¹-Gruppen zu ersetzen, die sich von OCH₃ unterscheiden.

## Claims

1. Process for the fixation or separation ions, which consists of contacting a medium containing said ions with a derivative of per(3,6-anhydro)cyclodextrin, complying with one of the following formulas: 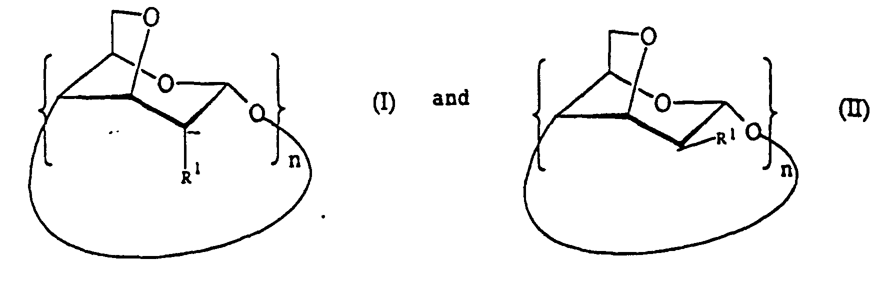 in which at least one of the R¹ represents the methoxy group and the other R¹, which can be the same or different, represent a group complying with one of the formulas: OH, OR², SH, SR², OCOR², NH₂, NR²R³, CONR²R³, CONH₂, CN, COOR², COOH and R², in which R² represents a saturated or unsaturated, aliphatic or aromatic, hydrocarbon group, which can have one or more heteroatoms chosen from among O, S and N, and R³ represents a hydrogen atom or a saturated or unsaturated, aromatic or aliphatic, hydrocarbon group, which can have one or more heteroatoms chosen from among O, S and N, and n is equal to 6, 7 or 8, for fixing said ions in complex form with the per(3,6-anhydro)cyclodextrin derivative and for separating them from said medium.

2. Process according to claim 1, wherein said ions are lead ions.

3. Process according to claim 1 or 2, wherein n is equal to 6.

4. Process according to claim 1 or 2, wherein the per(3,6-anhydro) cyclodextrin derivative complies with formula (I), in which all the R¹ represent the nethoxy group and n is equal to 6.

5. Process according to any one of the claims 1 to 4, wherein said medium is an aqueous solution, the cyclodextrin derivative being dissolved in an organic solvent immiscible with the aqueous solution.

6. Pharmaceutical composition for the lead decontamination of a living being, **characterized in that** it comprises a per(3,6-anhydro) cyclodextrin derivative complying with one of the following formulas: 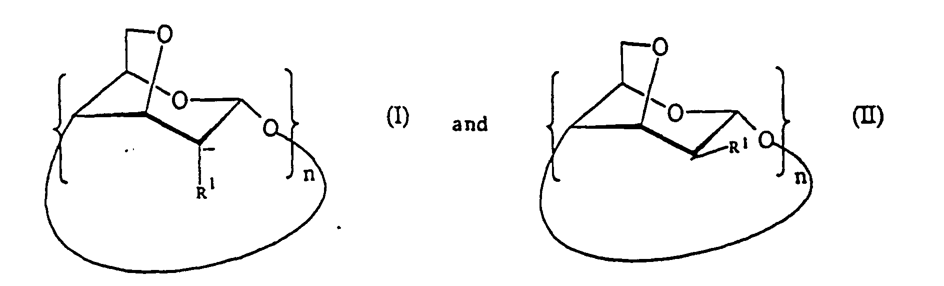 in which at least one of the R¹ represents the methoxy group and the other R¹, which can be the same or different, represent a group complying with one of the formulas: OH, OR², SH, SR², OCOR², NH₂, NR²R³, CONR²R³, CONH₂, CN, COOR², COOH and R², in which R² represents a saturated or unsaturated, aliphatic or aromatic, hydrocarbon group, which can have one or more heteroatoms chosen from among O, S and N, and R³ represents a hydrogen atom or a saturated or unsaturated, aliphatic or aromatic, hydrocarbon group, which can have one or more heteroatoms chosen from among O, S and N, and n is equal to 6, 7 or 8.

7. Pharmaceutical composition according to claim 6, wherein the per(3,6-anhydro)cyclodextrin derivative complies with formula (I), in which all the R¹ represent the methoxy group and n is equal to 6.

8. Complex of lead and a derivative of per(3,6-anhydro)cyclodextrin complying with one of the following formulas: 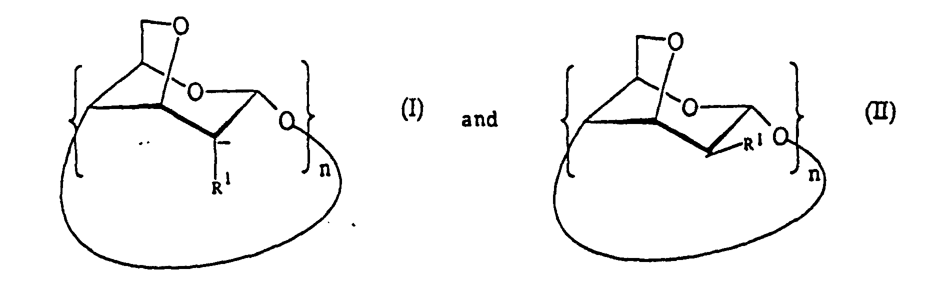 in which at least one of the R¹ represents the nethoxy group and the other R¹, which can be the same or different, represent a group complying with one of the formulas: OH, OR², SH, SR², OCOR², NH₂, NR²R³, CONR²R³, CONH₂, CN, COOR², COOH and R², in which R² represents a saturated or unsaturated, aliphatic or aromatic hydrocarbon group, which can have one or more heteroatoms chosen from among O, S and N, and R³ represents a hydrogen atom or a saturated or unsaturated, aliphatic or aromatic, hydrocarbon group, which can have one or more heteroatoms chosen from among O, S and N, and n is equal is 6, 7 or 8.

9. Complex according to claim 8, wherein n is equal to 6.

10. Complex according to claim 8, wherein the per(3,6-anhydro)cyclodextrin derivative complies with formula (I), in which all the R¹ represent the methoxy group and n is equal to 6.

11. Derivative of per(3,6-anhydro)cyclodextrin complying with one of the following formulas: 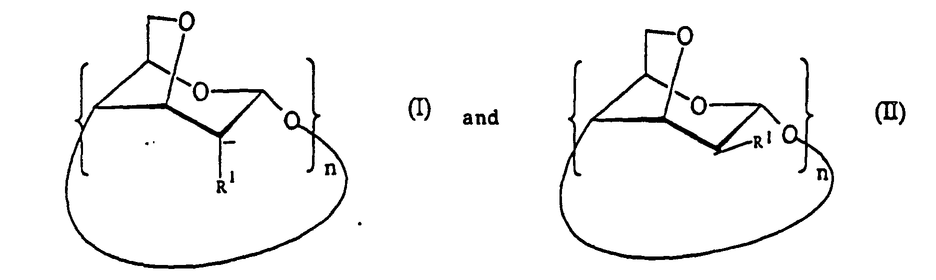 in which at least one of the R¹ represents the methoxy group and the other R¹, which can be the same or different, represent a group complying with one of the formulas: OH, OR², SH, SR², OCOR², NH₂, NR²R³, CONR²R³, CONH₂, CN, COOR², COOH and R², in which R² represents a saturated or unsaturated, aliphatic or aromatic, hydrocarbon group, which can have one or more heteroatoms chosen from among O, S and N, and R³ represents a hydrogen atom or a saturated or unsaturated, aliphatic or aromatic, hydrocarbon group, which can have one or more heteroatoms chosen from among O, S and N, and n is equal to 6, 7 or 8, provided that all the R¹ do not represent OCH₃ when n = 7 and that the derivative complies with formula (I).

12. Derivative of per(3,6-anhydro)cyclodextrin according to claim 11 complying with formula (I), in which all the R¹ represents the OCH₃ group and n is equal to 6.

13. Process for the preparation of a per(3,6-anhydro)cyclodextrin derivative of formula (I) or (II): 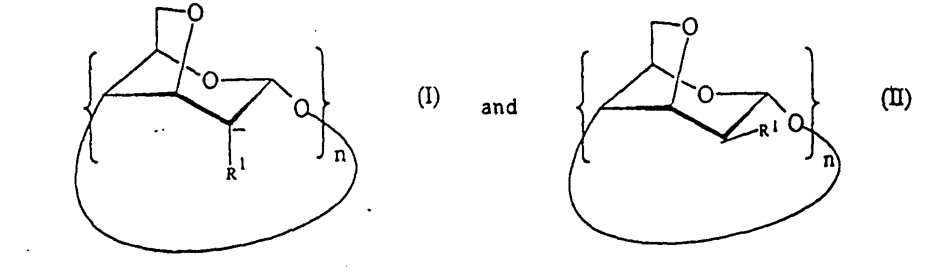 in which at least one of the R¹ represents the methoxy group and the other R¹, which can be the same or different, represent a group complying with one of the formulas: OH, OR², SH, SR², OCOR², NH₂, NR²R³, CONR²R³, CONH₂, CN, COOR², COOH and R², in which R² represents a saturated or unsaturated, aliphatic or aromatic, hydrocarbon group, which can have one or more heteroatoms chosen from among O, S and N, and R³ represents a hydrogen atom or a saturated or unsaturated, aliphatic or aromatic, hydrocarbon group, which can have one or more heteroatoms chosen from among O, S and N, and n is equal to 6, 7 or 8, comprising the following stages:
1) reacting a peranhydrocyclodextrin complying with one of the formulas: 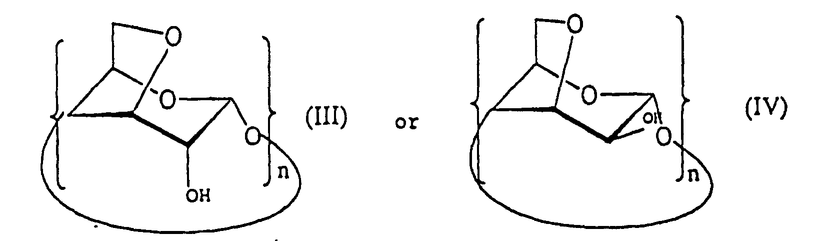 in which n is equal to 6, 7 or 8, with an alkali metal hydride for converting the OH group or groups into OM group or groups with M representing an alkali metal,
2) reacting the modified peranhydrocyclodextrin obtained in 1) with a methyl anhydride of formula CH₃X, in which X represents a halogen atom, and
3) if necessary, reacting the peranhydrocyclodextrin obtained in 2) with one or more reagents for substituting it by R¹ groups differing from OCH₃.
